(19) Europäisches Patentamt
European Patent Office
Office européen des brevets

(11) Publication number: **0 137 445**
**B1**

(12) # EUROPEAN PATENT SPECIFICATION

(45) Date of publication of patent specification: **03.01.90**

(51) Int. Cl.$^5$: **C 07 D 309/30**

(21) Application number: **84111760.9**

(22) Date of filing: **02.10.84**

(54) **Process for c-methylation of 2-methylbutyrates.**

(30) Priority: **11.10.83 US 540954**

(43) Date of publication of application:
**17.04.85 Bulletin 85/16**

(45) Publication of the grant of the patent:
**03.01.90 Bulletin 90/01**

(84) Designated Contracting States:
**CH DE FR GB IT LI NL**

(56) References cited:
**EP-A-0 030 528**
**EP-A-0 033 537**
**EP-A-0 033 538**
**EP-A-0 094 443**
**US-A-3 983 140**

**The file contains technical information submitted after the application was filed and not included in this specification**

(73) Proprietor: **MERCK & CO. INC.**
**126, East Lincoln Avenue P.O. Box 2000**
**Rahway New Jersey 07065-0900 (US)**

(72) Inventor: **Sletzinger, Meyer**
**135 Rockview Avenue**
**North Plainfield New Jersey 07060 (US)**
Inventor: **Verhoeven, Thomas R.**
**106 Oak Lane**
**Cranford New Jersey 07016 (US)**
Inventor: **Volante, Ralph P.**
**22 Hawthorne Lane**
**East Windsor New Jersey 08520 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

**EP 0 137 445 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention is concerned with a novel process which may be depicted as:

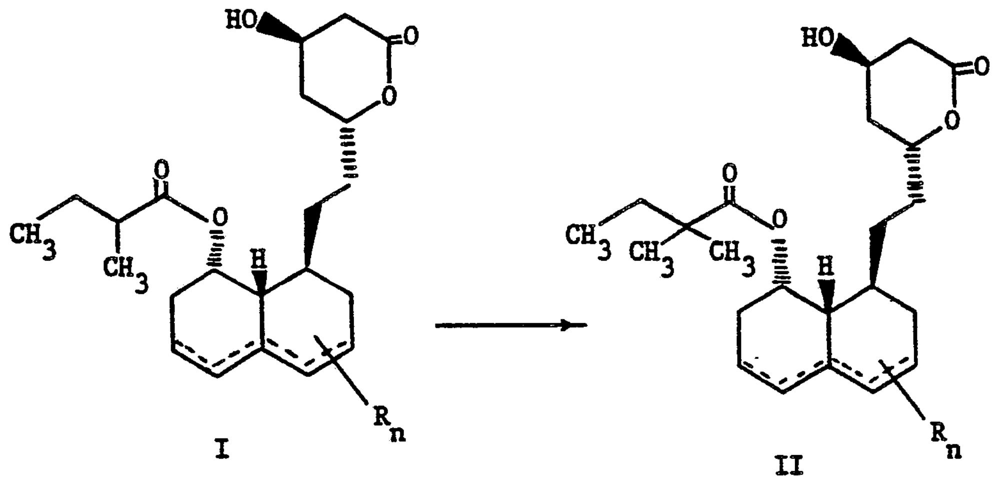

I II

The products II, of the process are more active inhibitors of HMG-CoA reductase than are the starting Compounds I, and thus of greater utility in the treatment of atherosclerosis, hyperlipemia, familial hypercholesterolemia and like disorders.

Background of the Invention

Compounds of structure I are known and known to have HMG-CoA reductase inhibitory properties. They are the natural fermentation products mevinolin (U.S. Patent 4,231,938) and compactin (U.S. Patent 3,983,140) and derivatives thereof, all with the natural 2-methylbutyrate side chain.

Hydrogenation products, including tetrahydromevinolin are described in U.S. Patent 4,351,844.

Some compounds of structure II with the 2,2-dimethylbutyrate side chain and processes for their preparation are known in EPO published application 33538. However, the process disclosed therein involves 4 distinct chemical steps: 1) de-esterification of the 2-methylbutyrate; 2) protection of the 4-hydroxy of the pyranone ring; 3) re-esterification to form the desired 2,2-dimethylbutyrate; and 4) deprotection of the 4-hydroxy group.

Now, with the present invention there is provided a novel process for the preparation of compounds of structure II involving only one chemical step and resulting in overall yields much higher than those realized by the prior art process, with the expenditure of much less time, labor and materials.

Detailed Description of the Invention

The novel process of this invention may be represented by:

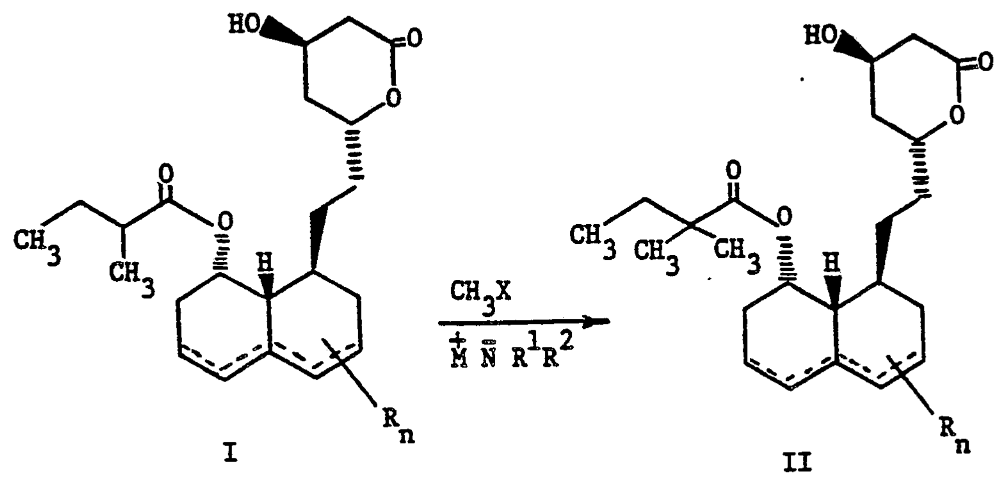


I II

wherein the dotted lines represent possible double bonds there being 0, 1 or 2 double bonds;

n represents 1, 2, 3 or 4; and

R is 1) methyl,

2) hydroxy, or

3) $C_{1-4}$alkoxy,
$R^1$ and $R^2$ are independently
1) $C_{1-3}$alkyl, or
2) $R^1$ and $R^2$ joined together, form a 5 or 6-membered heterocycle such as pyrrolidine or piperidine with the nitrogen to which they are attached;
X is halo, especially chloro, bromo or iodo; and
$\overset{+}{M}$ is a cation derived from lithium, sodium, or potassium.

A preferred use for the novel process of this invention is in the preparation of compounds of formula II wherein there is no double bond, one double bond in the 3,4-position, or two double bonds in the 3,4- and 4a,5 positions; n is 1 or 2; and R is methyl in the 2-position if n = 1 and in the 2 and 6-positions when n = 2.

A most preferred use for the novel process is in the preparation of the compound of structural formula:

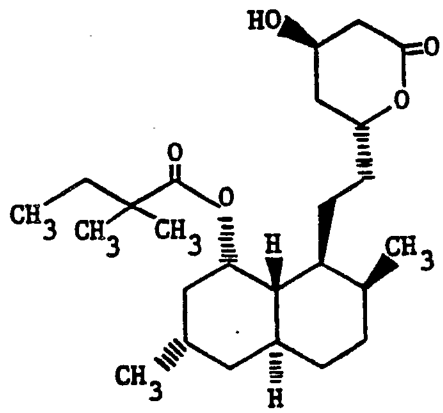

The novel process comprises C-methylation at the 2-position of the 2-methylbutyryloxy group of I at the 8-position of the polyhydronaphthalene moiety. The lactone compound is first converted to an alkali metal salt, preferably the potassium salt, of the dihydroxycarboxylate. Although any conceivable process for preparing a dry salt would suffice, it is convenient to add a substantially stoichiometric amount of aqueous potassium hydroxide to a solution of the lactone starting material in a hydrocarbon solvent such as benzene, toluene or cyclohexane containing a small amount of a $C_{1-3}$ alkanol, preferably isopropanol, ethanol or methanol, stirring for a few minutes to about an hour and finally concentrating to dryness *in vacuo*. The residue is subjected to rigorous drying such as by azeotropic distillation with cyclohexane or toluene, as the actual methylation procedure that follows proceeds properly only under rigorously anhydrous conditions.

Preferably, the anhydrous alkali metal salt is dissolved in an ethereal solvent such as tetrahydrofuran, diethyl ether, or 1,2-dimethoxyethane, cooled and treated with an excess of the alkali metal amide, wherein the alkali metal is lithium, sodium or potassium, preferably lithium, and the amide is preferably diethylamide, pyrrolidide, dimethyl amide or diisopropyl amide in an ethereal solvent in a dry inert environment. After about 2 to 8 hours, preferably about 2 hours at about −60 to −25°C, prefearably −35 to −30°C, a methylhalide, esp. methylbromide, methylchloride or methyl iodide, preferably methyl bromide or methyl iodide, is added to the mixture while maintaining the low temperature. Treatment with the alkali amide and methyl halide as described can be repeated if appreciable quantities of starting material remain. Preferably the reaction is conducted in an ethereal solvent at temperatures of −60 to −25°C and the methylhalide is methylbromide or methyliodide. More preferably the ethereal solvent is tetrahydrofuran, the temperature is −35 to −30°C, the alkali metal amide is lithium diethylamide or lithium pyrrolidide, and the methylhalide is methylbromide or methyliodide. After about 0.5 to about 3 hours, following final addition of methylhalide the reaction mixture can be quenched by adding it to an excess of water. To isolate the product the aqueous phase is adjusted to pH 3—6 with a strong mineral acid such as hydrochlorid, hydrobromic, sulfuric, phosphoric acid or the like. The aqueous phase is extracted with cyclohexane or toluene, dried, filtered, refluxed for 3—20 hours and finally concentrated, and filtered.

Example 1

Preparation of 6(R)-[2-[8(S)(2,2-dimethylbutyryloxy)-2(S),6(S)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one

A solution of *trans*-tetrahydro mevinolin (5 g, 12.25 mmol) in cyclohexane (100 ml) and isopropanol (12 ml) was prepared under nitrogen. An aqueous solution of potassium hydroxide (4.91 *M*, 2.5 ml, 12.27 mmol) was added in one portion and the two-phase mixture stirred for 0.5 hr. at ambient temperature. The mixture was concentrated via distillation (bath temperature 100°C). The vessel was recharged with 150 ml of cyclohexane and reconcentrated to a volume of 15 ml. The potassium carboxylate solution was diluted with tetrahydrofuran (35 ml) and cooled to −35°C.

A solution of pyrrolidine (3.6 ml, 43.1 mmol) in tetrahydrofuran (30 ml) was cooled to −5°C and a solution of n-butyllithium (27.5 ml, 42.6 mmol, 1.55 *M* in hexane) was gradually added maintaining an internal temperature below 0°C during the addition.

The lithium pyrrolidide thus prepared was added to the cooled solution of the potassium carboxylate via cannula, maintaining an internal temperature below −30°C throughout the addition. The clear yellow solution was aged between −35 to −30°C for 2 hours. A solution of methyl bromide (2.36, 24.8 mmol) in tetrahydrofuran was added maintaining an internal temperature of −20°C. The white slurry was aged for 1 hour at this temperature. A solution of pyrrolidine (1.6 ml, 19.16 mmol) in tetrahydrofuran (15 ml) was cooled to −5°C and n-butyllithium (12 ml, 18.6 mmol, 1.55 *M* in hexane) was added maintaining the temperature below 0°C. This solution was gradually added to the reaction mixture maintaining an internal temperature below −30°C. The mixture was aged at −30 to −35°C for 2 hours. A solution of methyl bromide (3.01 g, 31.7 mmol) in tetrahydrofuran was added maintaining an internal temperature of −20°C. The mixture was aged at that temperature for 1 hour.

The mixture was quenched into a vessel containing water (100 ml), the layers separated and the lower (aqueous) phase adjusted to pH 4.5 with 20% aqueous phosphoric acid. The acidified aqueous phase was extracted three times with 100 ml of cyclohexane. The combined cyclohexane extracts were washed twice with 50 ml of water, then dried over sodium sulfate (25 g). The mixture was filtered and slowly concentrated to a volume of 40 ml via distillation over 5 hours. After cooling to ambient temperature, the mixture was filtered to give crude product (4.15—4.25 g) of approximately 90—92% purity. The product was dissolved in methanol (22 ml/g of substrate) and water (6.2 ml/g) with stirring at 65°C while additional water (6.2 ml/g) was added. The mixture was aged at ambient temperature overnight, filtered and dried under vacuum at 50°C to yield pure product (85—92% recovery). Overall yield from *trans*-tetrahydro mevinolin is 67—75%.

Example 2

Alternate Preparation of 6(R)-[2-[8(S)(2,2-dimethyl-butyryloxy)-2(S),6(S)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one

A solution of *trans*-tetrahydro mevinolin (5 g, 12.25 mmol) in toluene (60 ml) and methanol (12 ml) was prepared in a 250 ml round bottom flask under nitrogen.

A titrated aqueous solution of potassium hydroxide (4.91 *M*, 2.5 ml, 12.27 mmol) was added in one portion and the two-phase mixture stirred for 0.5 hour at ambient temperature.

The mixture was concentrated to dryness on a rotary evaporator (bath temperature 60—75°C, 60—70 mm Hg). The vessel was recharged with 100 ml of toluene and reconcentrated. This recharge and concentration process was repeated providing a cream colored foam.

The dried potassium carboxylate was dissolved in tetrahydrofuran (45 ml) and cooled to −35°C.

A solution of diethylamine (4.5 ml, 43.5 mmol) in tetrahydrofuran (30 ml) was cooled to −25°C and a solution of n-butyllithium (27.5 ml, 42.6 mmol, 1.55 *M* in hexane) was gradually added maintaining an internal temperature below −20° during the addition.

The lithium diethylamide thus prepared was added to the cooled solution of the potassium carboxylate via cannula, maintaining an internal temperature below −30°C throughout the addition. The clear yellow solution was aged between −35 to 30°C for 5 hours.

Methyl iodide (1.4 ml, 22.5 mmol) was added dropwise maintaining an internal temperature of −30°C. The white slurry was aged for 1 hour at this temperature.

A solution of diethylamine (2 ml, 19.3 mmol) in tetrahydrofuran (15 ml) was cooled to −25°C and n-butyllithium (12 ml, 18.6 mmol, 1.55 *M* in hexane) was added, maintaining the temperature below −20°C. This solution was gradually added to the reaction mixture maintaining an internal temperature below −30°C. The mixture was aged at −30 to −35°C for 2 hours.

Methyl iodide (2 ml, 32.13 mmol) was added maintaining an internal temperature of −30°C. The mixture was aged at that temperature for 1 hour.

The mixture was quenched into a vessel containing toluene (200 ml) and water (100 ml).

The mixture was adjusted to pH 4.5 with 20% aqueous phosphoric acid (approx. 30 ml). The layers were separated and the upper (organic) phase stirred over sodium sulfate (25 g) and sodium bisulfite (1.5 g) for 2 hours. The mixture was filtered through Supercel[R] and transferred to a 500 ml round bottom flask, fitted with a distillation head and heated for 6—10 hours (bath temperature 110°C).

The toluene solution was concentrated to dryness to give crude product (5.3—5.4 g). Crude product was stirred in refluxing cyclohexane (50 ml) for 1 hour then allowed to cool with stirring to room temperature and aged an additional 1 hour, filtered and dried under vacuum at 50°C to yield product (3.4—3.8 g) of approximately 90—92% purity.

The product was dissolved in methanol (22.6 ml/g of substrate) and water (6.2 ml/g) with stirring at 65°C. The solution was filtered then heated at 65°C while additional water (6.2 ml/g) was added. The mixture was aged at ambient temperature overnight, filtered and dried under vacuum at 50°C with a nitrogen purge to yield pure product (85—92% recovery). Overall yield from *trans*-tetrahydro mevinolin is 58—62%.

Example 3

Alternate Preparation of 6(R)-[2-[8(S)(2,2-dimethyl-butyryloxy)-2-(S),6(S)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one

The potassium salt of *trans*-tetrahydromevinolin (20 g, 49 mmole) is prepared in cyclohexane (400 ml), isopropanol (48 ml) and aqueous potassium hydroxide (10 ml, 4.91 molar) as described above. The mixture is concentrated by distillation at atmospheric pressure. Additional cyclohexane (450 ml) is added. A total of 600 ml of distillate is collected. A KF of less than 70 µg $H_2O$/ml should be observed. The mixture is concentrated to a total volume of 52 ml.

Tetrahydrofuran (280 ml) and pyrrolidine (16 ml) is charged into the vessel and the solution cooled to less than −55°C. Butyllithium (110 ml, 1.55 *M*) is slowly added to the well stirred mixture, maintaining an internal temperature below −55°C throughout the addition. The mixture is aged at −30° to −35°C for 2.5 hours. Methylbromide (10.0 g) is bubbled into the solution maintaining an internal temperature of −20° to −25°C. After an age of 15 minutes HPLC analysis is performed to confirm greater than 93% conversion. If less than 93% conversion is observed a second charge of methyl bromide (normally 0.25—0.75 g) is introduced. The mixture is aged at −20° to −25°C for a total of one hour.

Tetrahydrofuran (60 ml) and pyrrolidine (6.4 ml) are charged to the reaction mixture and cooled to less than −55°C. n-BuLi (48 ml, 1.55 *M*) is slowly added as before maintaining an internal temperature below −55°C during the addition. The mixture is aged for two hours at −30° to −35°C. Methyl bromide (12 g) is introduced as described above and the mixture aged for 1 hour at −20° to −25°C. The reaction mixture is quenched into 400 ml of $H_2O$ and worked up and relactonized as described above. The crude yield after filtration of cyclohexane slurry and drying (40°C *in vacuo*) 17.89 g, 86.9% pure. Overall yield is 75.2%.

Employing the procedure substantially as described in Examples 1 and 2, but substituting for the *trans*-tetrahydromevinolin used as starting material therein, approximately equimolecular amounts of the 2-methylbutyrates described in Table I, there are prepared the 2,2-dimethylbutyrate products, also described in Table I:

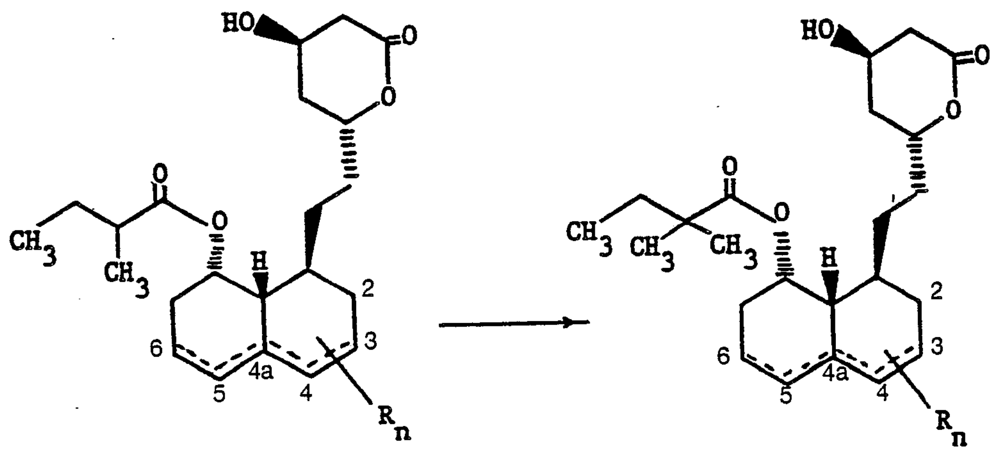

TABLE I

| Double Bonds | Rn |
|---|---|
| 3,4:4a,5 | 2-$CH_3$, 6-$CH_3$ |
| 3,4 | 2-$CH_3$, 6-$CH_3$ |
| 4,4a | 2-$CH_3$, 6-$CH_3$ |
| 4a,5 | 2-$CH_3$, 6-$CH_3$ |
| 3,4:4a,5 | 2-$CH_3$ |
| 3,4:4a,5 | 2-$CH_3$ |
| 3,4 | 2-$CH_3$ |
| — | 2-$CH_3$ |
| 3,4:4a,5 | 2-$CH_3$, 6-OH |
| 4,4a | 2-$CH_3$, 3-OH, 5-OH |

TABLE I continued

| Double Bonds | Rn |
|---|---|
| 4,4a:5,6 | 2-$CH_3$, 3-OH |
| 4,4a | 2-$CH_3$ |
| 4a,5 | 2-$CH_3$ |
| — | 2-$CH_3$, 6-OH |
| — | 2-$CH_3$, 3-OH |
| 4,4a | 2-$CH_3$, 6-OH |
| 4,4a | 2-$CH_3$, 3-OH |
| 4a,5 | 2-$CH_3$, 6-OH |
| 4a,5 | 2-$CH_3$, 3-OH |
| 4,4a:5,6 | 2-$CH_3$, 3-$OCH_3$ |
| — | 2-$CH_3$, 3-OH, 5-OH |
| 4.4a | 2-$CH_3$, 3-Cl, 5-Cl |
| 4.4a | 2-$CH_3$, 3-$OC_2H_5$, 5-OH |
| 4.4a | 2-$CH_3$, 3-$OC_4H_9$, 5-OH |
| 4.4a | 2-$CH_3$, 6-$CH_3$, 3-OH, 5-OH |

**Claims**

1. A process for the preparation of a compound of structural formula II:

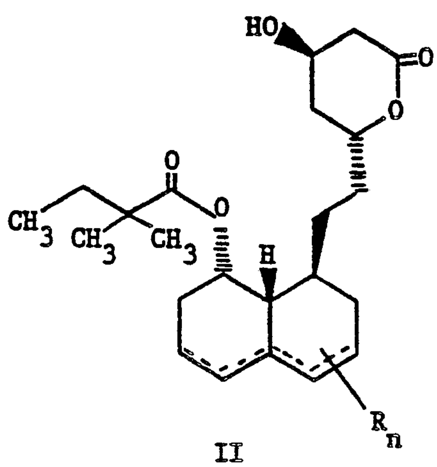

II

which comprises treatment of an anhydrous alkali metal salt of a compound of structural formula I

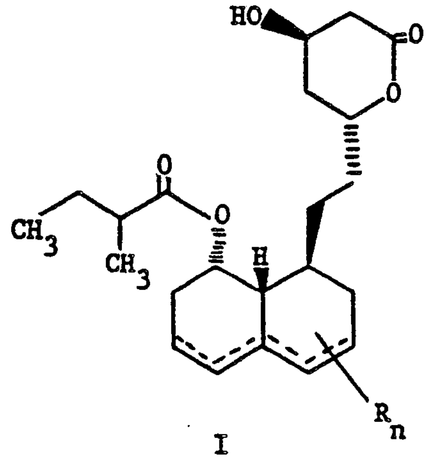

I

with a methyl halide and an alkali metal amide of formula

$$\overset{+}{M}\overset{-}{N}R^1R^2$$

where $\overset{+}{M}$ is a cation derived from sodium, potassium or lithium and $R^1$ and $R^2$ are $C_{1-3}$alkyl or $R^1$ and $R^2$ are joined together to form a 5 or 6-membered heterocyclic ring with the nitrogen to which they are attached, followed by acidification and lactonization wherein the dotted lines represent possible double bonds there being 0, 1 or 2 double bonds;

n represents 1, 2, 3 or 4; and

R is 1) methyl,

2) hydroxy, or

3) $C_{1-4}$alkoxy.

2. The process of Claim 1 which comprises treatment of Compound I with methyl iodide or methyl bromide in an ethereal solvent at −60°C to −25°C.

3. The process of Claim 2 wherein the ethereal solvent is tetrahydrofuran, the temperature is −35 to −30°C and the alkali metal amide is lithium diethylamide or lithium pyrrolidide.

4. The process of Claim 1 wherein in Compounds I and II there is no double bond, one double bond in the 3,4-position, or two bonds in the 3,4 and 4a,5-positions; n is 1 or 2; and R is methyl in the 2-position if n = 1 and in the 2 and 6-positions if n = 2.

5. The process of Claim 2 wherein in Compounds I and II there is no double bond, one double bond in the 3,4-position, or two bonds in the 3,4 and 4a,5-positions; n is 1 or 2; and R is methyl in the 2-position if n = 1 and in the 2 and 6-positions if n = 2.

6. The process of Claim 3 wherein in Compounds I and II there is no double bond, one double bond in the 3,4-position, or two double bonds in the 3,4 and 4a,5-positions; n is 1 or 2; and R is methyl in the 2-position if n = 1 and in the 2 and 6-positions if n = 2.

7. The process of Claim 1 for the preparation of the compound of formula:

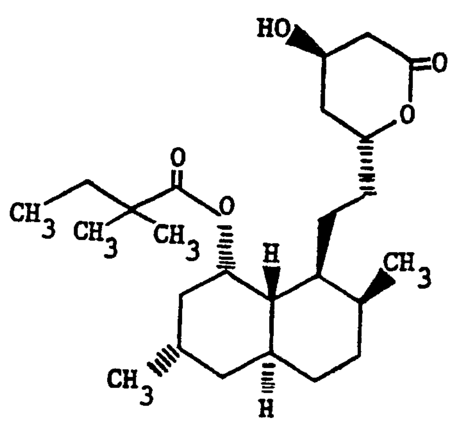

8. The process of Claim 2 for the preparation of the compound of formula:

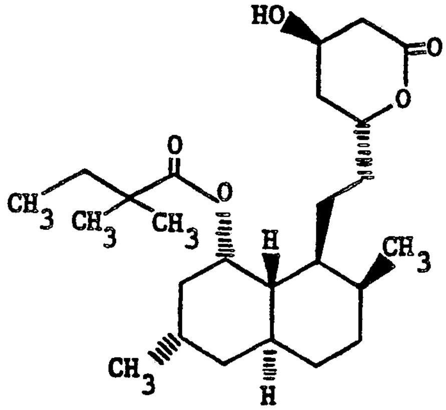

9. The process of Claim 3 for the preparation of the compound of formula:

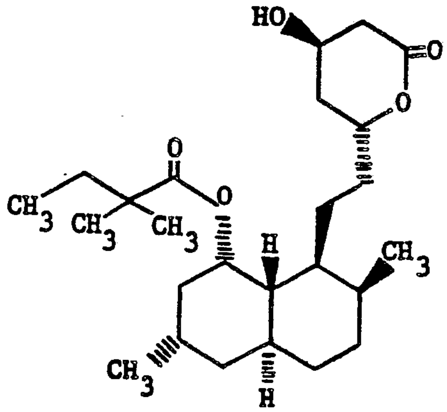

**Patentansprüche**

1. Ein Verfahren für die Herstellung einer Verbindung der Strukturformel II:

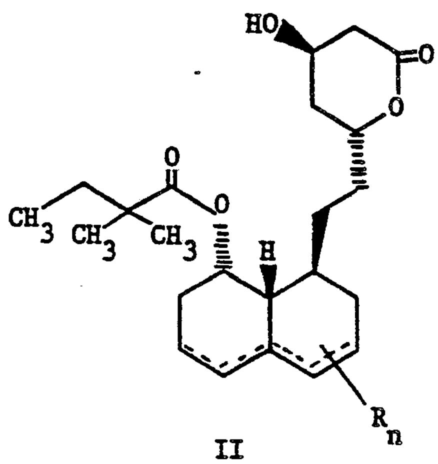

II

das umfasst die Behandlung eines wasserfreien Alkalimetallsalzes einer Verbindung der Strukturformel I

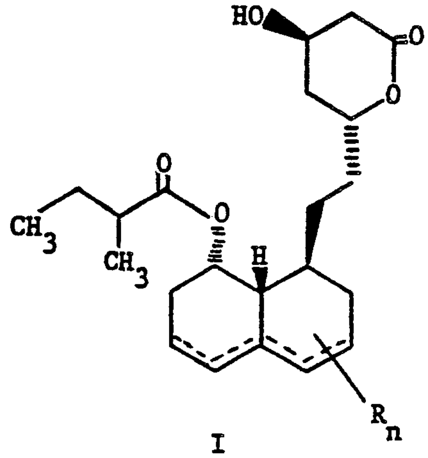

I

mit einem Methylhalogenid und einem Alkalimetallamid der Formel

$$\overset{+}{M}\overset{-}{N}R^1R^2,$$

worin $\overset{+}{M}$ ein von Natrium, Kalium oder Lithium abgeleitetes Kation ist und $R^1$ und $R^2$ $C_{1-3}$-Alkyl sind oder $R^1$ und $R^2$ miteinander verbunden sind und einen 5- oder 6-gliedrigen heterocyclischen Ring mit dem Stickstoff, an den sie gebunden sind, bilden, gefolgt von der Ansäuerung und der Lactonisierung, worin die gepunk-teten Linien mögliche Doppelbindungen darstellen, wobei 0, 1 oder 2 Doppelbindungen vorhanden sind;

n 1, 2, 3 oder 4 darstellt und

R 1) Methyl,

2) Hydroxy oder

3) $C_{1-4}$-Alkoxy ist.

2. Das Verfahren nach Anspruch 1, das die Behandlung der Verbindung I mit Methyljodid oder Methylbromid in einem etherischen Lösungsmittel bei −60° bis −25°C umfasst.

3. Das Verfahren nach Anspruch 2, worin das etherische Lösungsmittel Tetrahydrofuran, die Temperatur −35 bis −30°C und das Alkalimetallamid Lithiumdiethylamid oder Lithiumpyrrolidid sind.

4. Das Verfahren nach Anspruch 1, worin in den Verbindungen I und II keine Doppelbindung ist, eine Doppelbindung in der 3,4-Position ist, oder zwei Bindungen in den 3,4- und 4a,5-Positionen sind; n 1 oder 2 ist und R Methyl in der 2-Position, falls n = 1, and in den 2- and 6-Positionen, falls n = 2, ist.

5. Das Verfahren nach Anspruch 2, worin in den Verbindungen I und II keine Doppelbindung ist, eine Doppelbindung in der 3,4-Position ist, oder zwei Bindungen in den 3,4- und 4a,5-Positionen sind; n 1 oder 2 ist und R Methyl in der 2-Position, falls n = 1, und in den 2- und 6-Positionen, falls n = 2, ist.

6. Das Verfahren nach Anspruch 3, worin in den Verbindungen I und II keine Doppelbindung ist, eine Doppelbindung in der 3,4-Position ist, oder zwei Doppelbindungen in den 3,4- und 4a,5-Positionen sind; n 1 oder 2 ist und R Methyl in der 2-Position, falls n = 1, und in den 2- und 6-Positionen, falls n = 2, ist.

7. Das Verfahren nach Anspruch 1 für die Herstellung einer Verbindung der Formel:

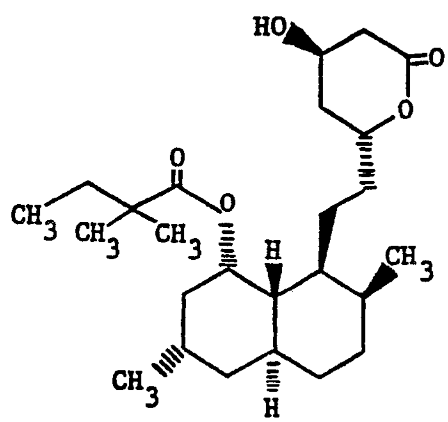

8. Das Verfahren nach Anspruch 2 für die Herstellung einer Verbindung der Formel:

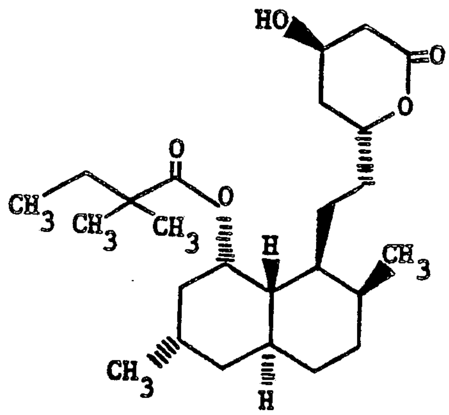

9. Das Verfahren nach Anspruch 3 für die Herstellung einer Verbindung der Formel:

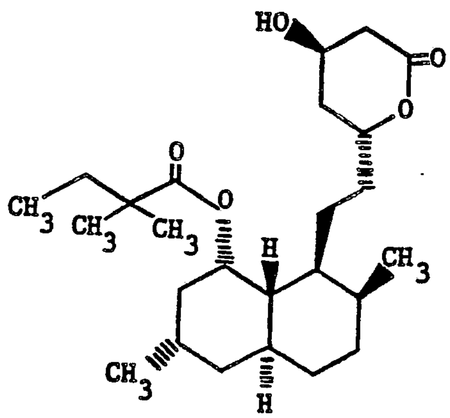

**Revendications**

1. Procédé pour la préparation d'un composé de formule structurelle II:

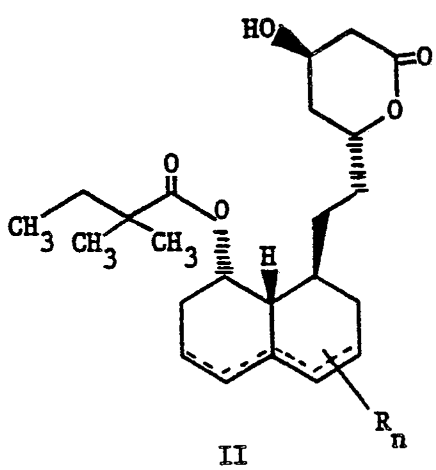

II

qui comprend le traitement d'un sel de métal alcalin anhydre d'un composé de formule structurelle I

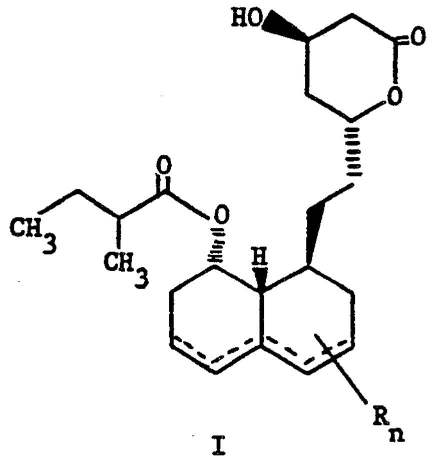

I

avec un halogénure de méthyle et un amidure de métal alcalin de formule $M^+N^-R^1R^2$ dans laquelle $M^+$ est un cation dérivé du sodium, du potassium ou du lithium et $R^1$ et $R^2$ sont une alkyle en $C_{1-3}$, ou $R^1$ et $R^2$ sont réunis ensemble pour former un cycle hétérocyclique à 5 ou 6 membres avec l'azote auquel ils sont attachés, suivi d'une acidification et d'une lactonisation, dans laquelle les lignes en pointillés représentent d'éventuelles doubles liaisons, avec la présence de 0,1 ou 2 doubles liaisons;

n représente 1, 2, 3 ou 4; et

R est 1) un méthyle,

2) un hydroxy, ou

3) un alcoxy en $C_{1-4}$.

2. Procédé de la revendication 1 comportant le traitement du composé I avec l'iodure de méthyle ou le bromure de méthyle dans un solvant éthéré, de −60° à −25°C.

3. Procédé de la revendication 2 dans lequel le solvant éthéré est le tétrahydrofuranne, la température est de −35 à −30°C et l'amidure de métal alcalin est le diéthylamidure de lithium ou le pyrrolidure de lithium.

4. Procédé de la revendication 1 dans lequel dans les composés I et II il n'y a pas de double liaison, il y a une double liaison dans la position 3,4, ou deux liaisons dans les positions 3,4 et 4a,5; n est 1 ou 2; et R est le méthyle dans la position 2 si n = 1 et dans les positions 2 et 6 si n = 2.

5. Procédé de la revendication 2 dans lequel, dans les composés I et II il n'y a pas de double liaison, il y a une double liaison dans la position 3,4, ou deux liaisons dans les positions 3,4 et 4a,5; n est 1 ou 2; et R est le méthyle dans la position 2 si n = 1 et dans les positions 2 et 6 si n = 2.

6. Procédé de la revendication 3 dans lequel, dans les composés I et II, il n'y a pas de double liaison, il y a une double liaison dans la position 3,4 et deux doubles liaisons dans les positions 3,4 et 4a,5; n est 1 ou 2; et R est le méthyle dans la position 2 si n = 1, et dans les positions 2 et 6 si n = 2.

7. Procédé de la revendication 1 pour la préparation du composé de formule:

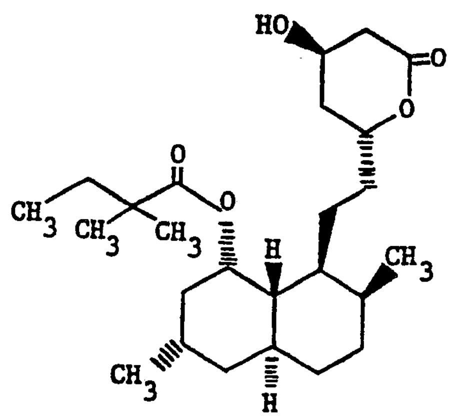

8. Procédé de la revendication 2 pour la préparation du composé de formule:

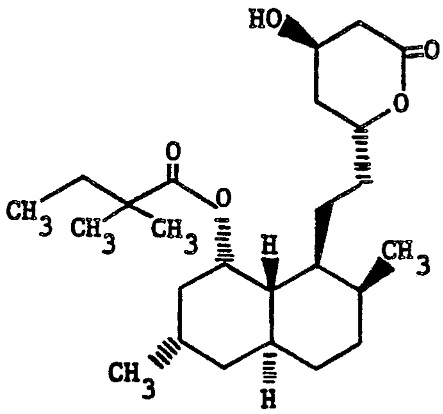

9. Procédé de la revendication 3 pour la préparation du composé de formule: